# EUROPEAN PATENT APPLICATION

(11) **EP 1 364 578 A1**
(43) Date of publication of application: **26.11.2003**
(21) Application number: 02700795.4
(22) Date of filing: 26.02.2002
(51) Int. Cl.: A01K 67/027, C12N 5/10, C12N 15/09, G01N 33/15, G01N 33/50

(54) **SCHIZOPHRENIA-LIKE MENTAL DISEASE MODEL ANIMAL, METHOD OF CONSTRUCTING THE SAME AND USE THEREOF**

(30) Priority: 27.02.2001 JP 2001052546
(71) Applicant: Japan as represented by President of Niigata University, Niigata-shi, Niigata 950-2181 (JP); Dainippon Pharmaceutical Co., Ltd., Osaka-shi, Osaka 541-8524 (JP)
(72) Inventor: NAWA, Hiroyuki, Niigata-shi, Niigata 951-8104 (JP); OKA, Makoto, Ibaraki-shi, Osaka 567-0011 (JP)
(74) Representative: von Kreisler, Alek, Dipl.-Chem.
(86) International application number: JP0201734
(87) International publication number: WO02067668

(57) **Abstract**

The present invention relates to a schizophrenia-like cognitive dysfunction animal model; a method of preparing the same; and a method of evaluating schizophrenia-like cognitive dysfunction by using said model. Specifically, the present invention relates to the preparation of a mammal showing continuous cognitive abnormality after sexual maturation, by allowing an excessive presence of interleukin 1 or an analog thereof and/or an intracellular signal transducer induced by interleukin 1 or an analog thereof in the body of young animal during the stages of brain function development; and a method of evaluating cognitive dysfunction by ethologically measuring the cognitive abnormality in the animal.

SEQUENCE LISTING

<110> President of Niigata University
      Dainippon Pharmaceutical Co., Ltd.

<120> Schizophrenia-like psychopathy animal model, production method
      thereof and use thereof.

<130> 09458

<150> 2001- 52546
<151> 2001-2-27

<160> 4

<170> PatentIn Ver. 2.1

<210> 1
<211> 480
<212> DNA
<213> Homo sapiens

<220>
<221> CDS
<222> (1)..(477)

<400> 1
tca tca cct ttt agc ttc ctg agc aat gtg aaa tac aac ttt atg agg      48
Ser Ser Pro Phe Ser Phe Leu Ser Asn Val Lys Tyr Asn Phe Met Arg

1                   5                   10                  15

atc atc aaa tac gaa ttc atc ctg aat gac gcc ctc aat caa agt ata     96

Ile Ile Lys Tyr Glu Phe Ile Leu Asn Asp Ala Leu Asn Gln Ser Ile

                20                  25                  30

att cga gcc aat gat cag tac ctc acg gct gct gca tta cat aat ctg     144

Ile Arg Ala Asn Asp Gln Tyr Leu Thr Ala Ala Ala Leu His Asn Leu

            35                  40                  45

gat gaa gca gtg aaa ttt gac atg ggt gct tat aag tca tca aag gat     192

Asp Glu Ala Val Lys Phe Asp Met Gly Ala Tyr Lys Ser Ser Lys Asp

        50                  55                  60

gat gct aaa att acc gtg att cta aga atc tca aaa act caa ttg tat     240

Asp Ala Lys Ile Thr Val Ile Leu Arg Ile Ser Lys Thr Gln Leu Tyr

65                  70                  75                  80

gtg act gcc caa gat gaa gac caa cca gtg ctg ctg aag gag atg cct     288

Val Thr Ala Gln Asp Glu Asp Gln Pro Val Leu Leu Lys Glu Met Pro

                85                  90                  95

gag ata ccc aaa acc atc aca ggt agt gag acc aac ctc ctc ttc ttc     336

Glu Ile Pro Lys Thr Ile Thr Gly Ser Glu Thr Asn Leu Leu Phe Phe

            100                 105                 110

tgg gaa act cac ggc act aag aac tat ttc aca tca gtt gcc cat cca     384

Trp Glu Thr His Gly Thr Lys Asn Tyr Phe Thr Ser Val Ala His Pro

        115                 120                 125

aac ttg ttt att gcc aca aag caa gac tac tgg gtg tgc ttg gca ggg     432

Asn Leu Phe Ile Ala Thr Lys Gln Asp Tyr Trp Val Cys Leu Ala Gly

    130                 135                 140

ggg cca ccc tct atc act gac ttt cag ata ctg gaa aac cag gcg tag     480

Gly Pro Pro Ser Ile Thr Asp Phe Gln Ile Leu Glu Asn Gln Ala

145                 150                 155

<210> 2

<211> 159

<212> PRT

<213> Homo sapiens

<400> 2

Ser Ser Pro Phe Ser Phe Leu Ser Asn Val Lys Tyr Asn Phe Met Arg

1               5                   10                  15

Ile Ile Lys Tyr Glu Phe Ile Leu Asn Asp Ala Leu Asn Gln Ser Ile

            20                  25                  30

Ile Arg Ala Asn Asp Gln Tyr Leu Thr Ala Ala Ala Leu His Asn Leu

        35                  40                  45

Asp Glu Ala Val Lys Phe Asp Met Gly Ala Tyr Lys Ser Ser Lys Asp

    50                  55                  60

Asp Ala Lys Ile Thr Val Ile Leu Arg Ile Ser Lys Thr Gln Leu Tyr

65                  70                  75                  80

Val Thr Ala Gln Asp Glu Asp Gln Pro Val Leu Leu Lys Glu Met Pro

                85                  90                  95

Glu Ile Pro Lys Thr Ile Thr Gly Ser Glu Thr Asn Leu Leu Phe Phe

            100                 105                 110

Trp Glu Thr His Gly Thr Lys Asn Tyr Phe Thr Ser Val Ala His Pro

        115                 120                 125

Asn Leu Phe Ile Ala Thr Lys Gln Asp Tyr Trp Val Cys Leu Ala Gly

    130                 135                 140

Gly Pro Pro Ser Ile Thr Asp Phe Gln Ile Leu Glu Asn Gln Ala

145                 150                 155

<210> 3

<211> 462

<212> DNA

<213> Homo sapiens

<220>

<221> CDS

<222> (1)..(459)

<400> 3

gca cct gta cga tca ctg aac tgc acg ctc cgg gac tca cag caa aaa 48

Ala Pro Val Arg Ser Leu Asn Cys Thr Leu Arg Asp Ser Gln Gln Lys

1               5                   10                  15

agc ttg gtg atg tct ggt cca tat gaa ctg aaa gct ctc cac ctc cag 96

Ser Leu Val Met Ser Gly Pro Tyr Glu Leu Lys Ala Leu His Leu Gln

            20                  25                  30

gga cag gat atg gag caa caa gtg gtg ttc tcc atg tcc ttt gta caa 144

Gly Gln Asp Met Glu Gln Gln Val Val Phe Ser Met Ser Phe Val Gln

        35                  40                  45

gga gaa gaa agt aat gac aaa ata cct gtg gcc ttg ggc ctc aag gaa 192

Gly Glu Glu Ser Asn Asp Lys Ile Pro Val Ala Leu Gly Leu Lys Glu

    50                  55                  60

aag aat ctg tac ctg tcc tgc gtg ttg aaa gat gat aag ccc act cta 240

Lys Asn Leu Tyr Leu Ser Cys Val Leu Lys Asp Asp Lys Pro Thr Leu

65                  70                  75                  80

cag ctg gag agt gta gat ccc aaa aat tac cca aag aag aag atg gaa 288

Gln Leu Glu Ser Val Asp Pro Lys Asn Tyr Pro Lys Lys Lys Met Glu

                85                  90                  95

aag cga ttt gtc ttc aac aag ata gaa atc aat aac aag ctg gaa ttt 336

Lys Arg Phe Val Phe Asn Lys Ile Glu Ile Asn Asn Lys Leu Glu Phe

            100                 105                 110
gag tct gcc cag ttc ccc aac tgg tac atc agc acc tct caa gca gaa 384
Glu Ser Ala Gln Phe Pro Asn Trp Tyr Ile Ser Thr Ser Gln Ala Glu
        115                 120                 125
aac atg ccc gtc ttc ctg gga ggg acc aaa ggc ggc cag gat ata act 432
Asn Met Pro Val Phe Leu Gly Gly Thr Lys Gly Gly Gln Asp Ile Thr
    130                 135                 140
gac ttc acc atg caa ttt gtg tct tcc taa 462
Asp Phe Thr Met Gln Phe Val Ser Ser
145                 150

<210> 4
<211> 153
<212> PRT
<213> Homo sapiens

<400> 4
Ala Pro Val Arg Ser Leu Asn Cys Thr Leu Arg Asp Ser Gln Gln Lys
1               5                   10                  15
Ser Leu Val Met Ser Gly Pro Tyr Glu Leu Lys Ala Leu His Leu Gln
            20                  25                  30
Gly Gln Asp Met Glu Gln Gln Val Val Phe Ser Met Ser Phe Val Gln
        35                  40                  45
Gly Glu Glu Ser Asn Asp Lys Ile Pro Val Ala Leu Gly Leu Lys Glu
    50                  55                  60
Lys Asn Leu Tyr Leu Ser Cys Val Leu Lys Asp Asp Lys Pro Thr Leu
65                  70                  75                  80
Gln Leu Glu Ser Val Asp Pro Lys Asn Tyr Pro Lys Lys Lys Met Glu
                85                  90                  95

Lys Arg Phe Val Phe Asn Lys Ile Glu Ile Asn Asn Lys Leu Glu Phe
            100                 105                 110
Glu Ser Ala Gln Phe Pro Asn Trp Tyr Ile Ser Thr Ser Gln Ala Glu
        115                 120                 125
Asn Met Pro Val Phe Leu Gly Gly Thr Lys Gly Gly Gln Asp Ile Thr
    130                 135                 140
Asp Phe Thr Met Gln Phe Val Ser Ser
145                 150

## Description

### Technical Field

The present invention relates to a schizophrenia-like cognitive dysfunction animal model and a method of preparing the same as well as a method of evaluating the schizophrenia-like cognitive dysfunction using said animal, and a method of screening a therapeutic agent or a therapeutic method of said cognitive dysfunction.

### Background Art

Schizophrenia is an extremely serious chronic disease that 0.7-1.0% of the population develops, which has produced as many as hundreds of thousands of patients under long-term hospitalization in Japan. It starts in adolescence to late middle age and denotes characteristic symptoms in perception, thinking, feeling and behavior. In many cases it progresses chronically, and it is a mental disorder that gives rise to various difficulties in social adaptation. This disease accompanies various mental abnormalities including positive symptoms such as delusion, hallucination, auditory hallucination, attenuated thinking, catatonic symptom and strange behavior, and negative symptoms such as cognitive dysfunction including abnormal perception, loss of feeling, hypobulia, withdrawal and depressive symptom. In light of the unique pathology of this disease, establishment of a comprehensive treatment system including early diagnosis, treatment, activity for social rehabilitation and prevention of relapse has been desired. Nevertheless, even the biological disease state has not been understood clearly at present, much less the elucidation of its developmental etiology.

The only therapeutic agents useful for improving the positive symptoms of schizophrenia are considered to be the drugs that antagonize neurotransmitters: dopamine and serotonin. Specifically, phenothiazine compounds, thioxanthine compounds, butyrophenone compounds and benzamide compounds are frequently used for patients. In many cases, long-term administration of these drugs over many years is indispensable.

In addition to the studies of the action mechanism of these drugs, because psychostimulants such as amphetamine in fact induce positive symptoms of schizophrenia in human, a hypothesis has been proposed that an abnormal action of dopamine develops schizophrenia. Based on these facts, an animal under chronic administration of amphetamine is sometimes used as a schizophrenia model. Similarly, a drug having a hallucinogenic activity in human is also sometimes administered to an animal, which is then used as a schizophrenia model. An example thereof is an animal administered with phencyclidine which is an inhibitor of glutamic acid receptor considered to be involved in the physiological function of the brain in memory and learning.

Many of these conventional schizophrenia animal models show transient brain function abnormalities caused by drug administration and do not necessarily reproduce the disease state of chronic schizophrenia observed in human. In addition, the effect of dopamine antagonist is mainly an improvement in the positive symptoms, and therapeutic agents of negative symptoms are extremely limited. One of the major reasons therefor is considered to be the absence of a suitable schizophrenia animal model.

While a wide variety of hypotheses have indeed been proposed for the developmental mechanism of schizophrenia, inclusive of the above-mentioned abnormal dopamine action theory, among them is a neurodevelopmental hypothesis proposed by researchers principally involving Winberger (Weinberger DR., *Arch. Gen. Psychiatry, 44,* 660-669, 1987). It is, however, totally unknown as to what biological factors cause abnormality in the development of cerebral nerves by what mechanism.

There is a suggestion admitting the presence of a certain correlation between schizophrenia and interleukin 1 (hereinafter sometimes abbreviated as IL-1). For example, increase in IL-1 activity of schizophrenia patients (Sirota P., *Frog. Neurophychopharmacol. Biol. Psychiatry,* 19(1), 75-83, 1995), varying levels of IL-1β in schizophrenia patients (Barak *V., J. Basic Clin. Physiol. Pharmacol.,* 6(1), 61-69, 1995), a correlation between polymorphism of IL-1 gene and schizophrenia patients (Katila *H., Mol. Psychiatry, 4(2),* 179-81, 1999), an influence of psychotropic drugs on IL-1 receptor antagonists (Song C., *Schizophr. Res.,* 42(2), 157-64, 2000; Akiyama K., *schizophr. Res., 37(1),* 97-106, 1999) and the like have been known. As the present situation stands, however, whether the behavior of IL-1 actually has a causal association with the development of schizophrenia has not been clarified at all.

Some of other biological factors have been also suggested to relate to schizophrenia. For example, there are reports on the relationship between schizophrenia patients and neurotrophic factors (Takahashi M., *Molecular Psychiatry, 5,* 293-300, 2000), correlation between neurotrophic factors and cytokines in the development of nerves (Nawa H., *Molecular Psychiatry, 5(6),* 594-603, 2000), and the like. However, the actual causal association between these substances and schizophrenia has been unknown.

### Disclosure of the Invention

Accordingly, it is an object of the present invention to clarify a substance that inhibits the development of cerebral nerves to develop schizophrenia and artificially manipulate the behavior of said substance in the body, thereby to provide an experimental schizophrenia model that shows chronic cognitive ethological abnormalities extremely similar to those of schizophrenia in human. Another object of the present invention is to provide an evaluation method of schizophrenia-like cognitive dysfunction utilizing said animal model, and a screening method of an effective therapeutic agent of schizophrenia based on said evaluation method.

The present inventors have intensively studied with the aim of achieving the above-mentioned object and found that the excessive presence of IL-1 in young stages inhibits the brain functional development of animal and causes continuous schizophrenia-like cognitive dysfunction. That is, the present inventors have successfully prepared a schizophrenia-like cognitive dysfunction animal model showing continuous cognitive abnormality after sexual maturation, by administering IL-1 or an analog thereof to a young mammal during the stages of brain functional development, or introducing an expression vector comprising a nucleic acid encoding IL-1 or an analog thereof into a mammal or an embryonic cell thereof or the like, thereby allowing an excessive presence of IL-1 or an analog thereof in the body of said mammal during the entire or partial stages of brain development.

Moreover, the present inventors have established an evaluation method of cognitive dysfunction by performing various known cognitive ability tests with the schizophrenia-like cognitive dysfunction animal model obtained as described above and a method of screening a therapeutic agent of schizophrenia or a method of evaluating therapeutic method of schizophrenia, which comprises performing said evaluation method after the administration of a test substance or application of an external stimulus, which resulted in the completion of the present invention.

Accordingly, the present invention provides a mammal that is a schizophrenia-like cognitive dysfunction animal model characteristically showing continuous cognitive abnormality after sexual maturation, which is caused by an excessive presence of at least one of IL-1 or an analog thereof and/or intracellular signal transducers induced by IL-1 or an analog thereof in the body during the entire or partial stages of brain function development.

Said cognitive abnormality is recognized by the presence of a significant difference from the same kind of normal animal in conventionally known various ethological measurements such as prepulse inhibition in startle response, latent inhibition, social interaction and an amount of animal activity.

The schizophrenia-like cognitive dysfunction animal model of the present invention can be prepared by (1) administering at least one of IL-1 or an analog thereof and/or intracellular signal transducers induced by IL-1 or an analog thereof to a mammal in an amount sufficient to cause said cognitive abnormality during the entire or partial stages of brain function development, (2) introducing an expression vector comprising a nucleic acid encoding IL-1 or an analog thereof into a mammal for expressing IL-1 or an analog thereof in the body of said mammal in an amount sufficient to cause said cognitive abnormality during the entire or partial stages of brain function development, or (3) modifying an intracellular signaling system using genetic engineering procedures for inducing the production of endogenous intracellular signal transducer(s) induced by IL-1 or an analog thereof and allowing production of said endogenous intracellular signal transducer(s) in the body of said mammal in an amount sufficient to cause cognitive abnormality during the entire or partial stages of brain function development. Therefore, the present invention also provides a method for preparing a schizophrenia-like cognitive dysfunction animal model by the methods mentioned above.

Furthermore, the present invention provides a method of evaluating schizophrenia-like cognitive dysfunction by subjecting the above-mentioned schizophrenia-like cognitive dysfunction animal model to various ethological measurements such as prepulse inhibition in startle response, latent inhibition, social interaction and an amount of animal activity for testing for cognitive abnormality. By investigating a cognitive abnormality-improving effect by performing said evaluation method after administration of a candidate compound of a therapeutic agent of schizophrenia to said model or after application of an external stimulus as a non-drug therapy of schizophrenia to said model, an effective therapeutic agent or therapeutic method of schizophrenia can be found. Thus, the present invention also provides a method of screening a therapeutic agent of schizophrenia and a method of evaluating a therapeutic method of schizophrenia, which are based on the above-mentioned protocol.

### Brief Description of the Drawings

Fig. is a graph showing the intensity of startle response to a sound of 120 db at 60 days after birth (A) and 22 days after birth (B).

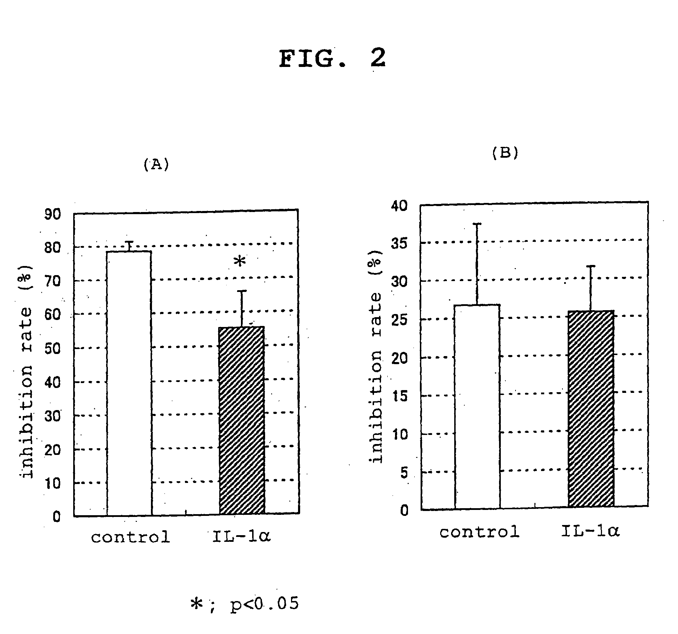

Fig. 2 is a graph showing the prepulse inhibition at 60 days after birth (A) and 22 days after birth (B).

### Detailed Description of the Invention

An animal to afford the animal model of the present invention is not particularly limited as long as it is a mammal, and includes, for example, mouse, rat, rabbit, guinea pig, monkey, pig, dog, cat and the like. In view of the fact that a lot of data have been accumulated so far and the transgenic techniques have been established, mouse, rat and monkey are more preferred. In addition, an inbred strain animal, whose genetic characteristics has been homogenized, is desirable, and in the case of an animal species for which SPF techniques have been established, the use of an SPF animal is more desirable.

The onset of continuous cognitive abnormality in the animal model of the present invention is attributable to the excessive presence of at least one of IL-1 or an analog thereof and/or intracellular signal transducers induced by IL-1 or an analog thereof in the body of animal during the entire or partial stages of brain function development. The stages of brain function development vary depending on the kinds of the mammal used. For example, in the case of mouse, the development of brain as defined by the weight almost completes in 20-30 days after birth. Thus, at least one of IL-1 or an analog thereof and/or intracellular signal transducers induced by IL-1 or an analog thereof should be present in excess in the body prior to the completion. Generally, such intracorporeal environment should be realized for the fetuses carried by female mouse or as early as possible after birth.

The term "interleukin 1 (IL-1)" used in the present invention generally means interleukin 1α (IL-1α) and interleukin 1β (IL-1β). It has been academically demonstrated that IL-1α and IL-1β bind to the same receptor and show similar biological activities. The IL-1 of the present invention is not limited as regards the origin, as long as it shows a biological activity similar to that of an endogenous IL-1 in the body of a mammal to be used, and includes, for example, IL-1α and IL-1β derived from human, monkey, pig, bovine, horse, dog, cat, mouse, rat, rabbit, hamster, guinea pig and the like. More specific example includes human IL-1α having an amino acid sequence represented by SEQ ID NO:2 and human IL-1β represented by SEQ ID NO:4. Furthermore, IL-1 analog is not particularly limited as long as it shows a biological activity similar to that of endogenous IL-1 in the body of a mammal to be used, and include, for example, a polypeptide consisting of an amino acid sequence wherein one or more amino acids are substituted, deleted, inserted; added or modified in the amino acid sequence of native IL-1α or IL-1β, and which have a biological activity similar to that of native IL-1.

IL-1 and an analog thereof may be produced by any method. For example, those recombinantly produced in large quantities in heterologous cells such as bacteria and yeast, those purified from animal cell and the like can be used.

IL-1 is one of cytokines, and is known to promote the activation of various intracellular signal transducers. The schizophrenia-like cognitive dysfunction in the animal model of the present invention is considered to be developed due to the action, as a second messenger, of an intracellular signal transducer produced excessively by the excessive presence of IL-1 or an analog thereof. Therefore, the animal model of the present invention can be prepared by artificially allowing an excessive presence or activation of these intracellular signal transducers. In the cerebral nerve system, for example, IL-1 induces activities of intracellular signaling factors, NFκB and IKK kinase in hypothalamus. Therefore, the "intracellular signal transducer(s) induced by IL-1 or an analog thereof" of the present invention includes these gene transcriptional factors and the like.

For administration or expression of IL-1 or an analog thereof, two methods are generally applicable. One of them is a method comprising directly injecting IL-1 or an analog thereof intraperitoneally or subcutaneously to an animal. According to this method, since the concentration of the exogenous IL-1 or an analog thereof in the body decreases soon due to the decomposition by metabolism, IL-1 or an analog thereof needs to be injected plural times for some days. For example, in the case of a mouse, a daily dose of 30-3,000 µg/kg may be administered for 1-20 days from 1-10 days after birth.

The other method comprises introducing an expression vector comprising a nucleic acid encoding IL-1 or an analog thereof to an individual animal to forcibly cause gene expression in the body of said animal, thereby to afford an effect similar to that achieved by the administration of said IL-1 or an analog thereof. This method is further classified into a method for transient expression of a transgene, and a method for preparation of a transgenic animal having said gene integrated in the animal chromosomes. In the latter method, once such transgenic animal is prepared and an inbred strain whose characteristics introduced has been genetically homogenized is established, the animal can be used repeatedly as a schizophrenia-like cognitive dysfunction animal model by simply maintaining the animal strain.

The "nucleic acid encoding IL-1 or an analog thereof" is not particularly limited as long as it encodes an amino acid sequence of the above-mentioned IL-1 or an analog thereof to be used in the present invention. For example, DNA sequence encoding an amino acid sequence of human IL-1α represented by SEQ ID NO:2 or an amino acid sequence of human IL-1β represented by SEQ ID NO:4, and human IL-1α coding sequence represented by SEQ ID NO:1 and human IL-1β coding sequence represented by SEQ ID NO:3, and DNA sequence that hybridizes with said DNA sequence under stringent condition, and which encodes a polypeptide showing a biological activity equivalent to that of IL-1 in the body of an animal or a protein having an amino acid sequence that binds to the same IL-1 receptor can be mentioned.

In IL-1 or an analog thereof (hereinafter sometimes abbreviated as IL-1s) expression vector of the present invention, the nucleic acid encoding the above-mentioned IL-1 or an analog thereof should be functionally linked to a promoter capable of showing a promoter activity in a cell of a target mammal, or should be positioned at a site where it can be converted to a functionally linked form under certain conditions in the animal cells into which it has been introduced. The promoter to be used is not particularly limited as long as it can function in a mammal cell, which is an administration subject, and includes, for example, virus promoters such as SV40-derived early promoter, cytomegalovirus LTR, Rous sarcoma virus LTR, MoMuLV-derived LTR and adenovirus-derived early promoter, and promoters of a gene of structural protein from a mammal, such as β-actin gene promoter, PGK gene promoter and transferrin gene promoter. By being "positioned at a site where it can be converted to a functionally linked form under certain conditions" is meant, for example, as explained in detail below, that the expression vector has a structure where a promoter and a nucleic acid encoding IL-1s are separated by two recombinase recognition sequences positioned in the same orientation, which are spaced apart by a spacer sequence having a length sufficient to prevent expression of IL-1s from said promoter, and that the spacer sequence is cleaved out in the presence of a recombinase that specifically recognizes said recognition sequences such that the nucleic acid encoding IL-1s is positioned to be functionally linked to the promoter.

Preferably, IL-1 expression vector of the present invention comprises a transcriptional termination signal (i.e. terminator region) in the downstream of a nucleic acid encoding IL-1s. Additionally, it preferably comprises a selection marker gene (gene which confers resistance to agent such as tetracycline, ampicillin, kanamycin, hygromycin and phosphinothricin, gene complementing auxotrophic mutation etc.) for transformant selection. When an expression vector has a spacer sequence between the recombinase recognition sequences as described above, said selection marker gene may be positioned within said spacer sequence.

A vector used as IL-1s expression vector of the present invention is not particularly limited. Preferable vectors for administration to mammals such as human include viral vectors such as retrovirus, adenovirus, adeno-associated virus, herpes virus, vaccinia virus, poxvirus, poliomyelitis virus, sindbis virus and sendai virus. Adenoviruses have advantages in that efficacy of gene introduction is extremely high, it can be introduced into a non-dividing cell, and the like. Furthermore, because integration of a transgene into host chromosome is extremely unusual, gene expression is transient, and generally lasts only for about 4 weeks. Therefore, it is particularly advantageous for transient excessive expression of IL-1 or an analog thereof in young animal during the stages of brain function development.

In a preferred embodiment of the present invention, IL-1s can be expressed from IL-1s expression vector in time-specific and brain tissue-specific manner, in order to prevent an adverse influence caused by an excessive expression of said IL-1s at unnecessary time and/or unnecessary site. In a first embodiment, such vector includes a vector comprising a nucleic acid encoding IL-1s, which is functionally linked to a promoter derived from a gene that specifically expresses in brain cell of an administration subject animal during the stages of brain function development. Those of ordinary skill in the art can easily select such conventionally known promoter.

In a second embodiment, a time-specific expression vector of the present invention includes a vector comprising a nucleic acid encoding IL-1s, which is functionally linked to an inducible promoter which regulates the expression in *trans* by an exogenous substance. For example, when a metallothionein-1 gene promoter is used as an inducible promoter, an inducible substance such as heavy metal such as gold, zinc and cadmium, steroid such as dexamethasone, alkylating agent, chelating agent and cytokine is administered to the body during the stages of brain function development, whereby the expression of IL-1s can be induced in a time-specific manner.

In a third embodiment, the time-specific expression vector of the present invention includes a vector having a structure where a promoter and a nucleic acid encoding IL-1s are spaced apart by a spacer sequence having a length sufficient to prevent expression of IL-1s from said promoter, and separated by two recombinase recognition sequences positioned in the same orientation. The promoter cannot direct transcription of IL-1s when the vector is simply introduced into animal cells. However, when recombinase which specifically recognizes said recognition sequence is administered or said recombinase is expressed in animal body by the administration of an expression vector comprising a nucleic acid encoding said recombinase during the stages of brain function development, homologous recombination occurs between said recognition sequences via said recombinase. As a result, said spacer sequence is excised, the nucleic acid encoding IL-1s is functionally linked to the promoter to generate IL-1s expression cassette, and IL-1s are expressed in a time-specific manner.

A recombinase recognition sequence to be used for the above-mentioned vector desirably has a heterogeneous recombinase recognition sequence, which is not recognized by an endogeneous recombinase in the administration subject, in order to prevent recombination with the endogeneous recombinase. Accordingly, the recombinase acting in *trans* on said vector is also preferably a heterologous recombinase. A preferred combination of such heterologous recombinase and said recombinase recognition sequence includes, but not limited to, Cre recombinase and lox P sequence derived from bacteriophage P1 in E. *coli,* and Flp recombinase and frt sequence derived from yeast.

While Cre recombinase was discovered in bacteriophage, it is known that specific DNA recombination reaction functions not only in prokaryotic cells, but also animal cells which are eukaryotic cells and animal viruses. When two loxP sequences exist on a single DNA molecule in the same orientation, DNA sequence between them is excised via Cre recombinase to form a cyclic molecule (excision reaction). When two loxP sequences exist on different DNA molecules and one of them is a cyclic DNA, the cyclic DNA is inserted into the other DNA molecule via loxP sequence (insertion reaction) [J. *Mol. Biol., 150:* 467-486 (1981); *J. Biol. Chem.,* 259: 1509-1514 (1984); *Proc. Natl. Acad. Sci. USA, 81:* 1026-1029 (1984)]. Examples of the excision reaction have been reported with regard to cultured animal cell *[Nucleic Acids Res., 17:* 147-161 (1989); *Gene, 181:* 207-212 (1996)], animal virus *[Proc. Natl. Acad. Sci. USA, 85:* 5166-5170 (1988); J. *Virol.,* 69: 4600-4606 (1995); *Nucleic. Acids Res.*, 23: 3816-3821 (1995)], transgenic mouse *[Proc. Natl. Acad. Sci. USA, 89:* 6232-6236 (1992); *Proc. Natl. Acad. Sci. USA, 89:* 6861-6865 (1992); *Cell*, *73:* 1155-1164 (1993); *Science,* 265:103-106 (1994)] and the like.

As a promoter of the time-specific IL-1 expression vector of the present invention utilizing an interaction between. recombinase and recombinase recognition sequence, a promoter derived from a virus or a promoter from the gene of a structure protein from mammal is preferably used to ensure the time-specific expression.

When a recombinase itself is administered as a trans-acting substance, for example, the recombinase may be dissolved or suspended in a suitable sterilized vehicle, and injected intraperitoneally, subcutaneously or the like.

When a recombinase expression vector is administered as a *trans*-acting substance, said recombinase expression vector is not particularly limited as long as it has an expression cassette where a nucleic acid encoding the recombinase is functionally linked to a promoter capable of showing the promoter activity in the cells of an animal which is an administration subject. When the promoter to be used is a constitutive promoter, the vector to be administered is desirably one that shows rare integration of the host cell and chromosome, such as adenovirus, so that the expression of recombinase can be prevented when the expression is not necessary. Another approach of expressing a recombinase when desired includes the use of inducible promoters such as metallothionein gene promoter.

Administration of IL-is expression vector for transient expression of IL-1 during the stages of brain function development is carried out either by an ex *vivo* method where the target cell (e.g., hematopoietic stem cell derived from bone marrow, peripheral blood and cord blood, lymphocyte, fibroblast etc.) is taken from the body, cultured and returned to the body by introduction, or an *in vivo* method where the vector is directly administered for introduction into the body of the administration subject. In the case of the *ex vivo* method, introduction of the vector into the target cell can be carried out by microinjection method, calcium phosphate coprecipitation method, PEG method, electroporation method or the like. In the case of the *in vivo* method, the viral vector can be administered intravenously, intraarterially, subcutaneously, intradermally, intramuscularly, intraperitoneally or the like in the form of an injection or the like. Alternatively, when a vector is administered by intravenous injection or the like, production of a neutralization antibody to said viral vector poses a problem. However, topical injection of the vector to the organ/tissue containing the target cell *(in situ* method) can reduce an adverse influence due to the presence of an antibody.

When the IL-1s expression vector of the present invention is a time-specific expression vector that utilizes an interaction between recombinase and recombinase recognition sequence, and a recombinase capable of *trans* reaction with said vector is administered in the form of a recombinase expression vector to the target tissue, the above-mentioned *in vivo* method can be used similarly as an administration method of the recombinase expression vector.

The animal model of the present invention can be also prepared as a transgenic animal introduced with IL-1s expression vector of the present invention. While any IL-1s expression vectors to be introduced which are mentioned above can be used, the use of a time-specific vector suitable for preparing a transgenic animal designed to express IL-1 only when needed in an amount sufficient to develop schizophrenia-like cognitive dysfunction in an animal, which is what is called a "conditional transgenic animal", is more preferable in consideration of the undesired influence during use thereof for the evaluation method of recognition dysfunction to be mentioned later and the like. In the present invention, a transgenic animal introduced with an expression vector comprising a nucleic acid encoding IL-1s under the regulation of inducible promoter or time-specific and/tissue-specific promoter is also encompassed in the "conditional transgenic animal".

The transgenic animal of the present invention can be prepared by introducing IL-1s expression vector of the present invention into a germ line cell of animal according to conventionally known transgenic production methods. For example, an oviduct of female after mating is washed, and a fertilized ovum is taken. Said vector is directly injected into a pronucleus derived from sperm or ovum. The fertilized ovum is transplanted into an oviduct of a pseudopregnant foster parent, and is allowed to grow continuously in uterus. Integration of the injected vector into the chromosomal DNA can be determined by screening a chromosomal DNA separated and extracted from the tail of an offspring with southern hybridization or PCR method.

When using mouse, hamster, pig and the like, a transgenic animal can be also obtained by introducing said vector into embryonic stem cell (ES cell). ES cell refers to a cell which is derived from an inner cell mass (ICM) of fertilized ovum in the blastocyst stage, and which can be cultured and maintained *in vitro* while keeping the undifferentiated state. ICM cell forms an embryo itself in the future, and is the origin of all tissues including germ cell. ES cell can be prepared as follows. A blastocyst is separated from a female after mating and cultured in a Petri dish. Then, partial cells of the blastocyst gather to form ICM, which is differentiated into embryo in the future. This inner cell mass is treated with trypsin to allow release of single cell to give ES cell. For introduction of gene into ES cell, transfection method, infection method using retroviral vector, electroporation method or the like is used. The best advantage of a system using ES cell is that transformant can be selected using a selection marker such as antibiotics resistance during the cell stage. When ES cell selected (i.e. transgene has been integrated) is microinjected into a blastocyst, it is internalized into ICM and a chimera embryo is generated. By transplanting this into a foster parent, and allowed to develop further, whereby a chimera transgenic animal is obtained. Individuals whose gonads are derived from ES cell are mated to prepare a transgenic animal whose characteristics recombined has been genetically homogenized.

Similarly, the animal model of the present invention can be prepared by directly injecting the above-mentioned intracellular signal transducer induced by IL-1 or an analog thereof intraperitoneally, subcutaneously or the like to a young animal during the stages of brain function development. In this case, again, exogenous intracellular signal transducer(s) needs to be injected plural times for some days, because the concentration thereof in the body decreases soon due to the decomposition by metabolism.

Furthermore, the animal model of the present invention can be also prepared by modifying intracellular signaling system using a genetic engineering method for inducing production of the above-mentioned intracellular signal transducer(s) induced by IL-1 or an analog thereof, thereby allowing young animal in the stages of brain function development to produce said endogenous intracellular signal transducer(s) in the body. For example, a method Comprising introduction of an expression vector comprising a nucleic acid encoding a peptide which promotes production of intracellular signal transducer(s) in trans, or alternatively a nucleic acid encoding a peptide that blocks activities of regulating factors which inhibit production of such transducer, into animal body to allow expression, using a method similar to that for the above-mentioned IL-1s expression vector can be mentioned.

An animal having mutation in the intracellular signal system such that endogenous intracellular signal transducer(s) induced by IL-1 or an analog thereof is excessively produced can be also prepared by artificially inducing mutagenesis in the animal cell.

The schizophrenia-like cognitive dysfunction animal model of the present invention, which is prepared by any of the above-mentioned methods, characteristically shows a particularly remarkable symptom of continuous cognitive abnormality after sexual maturation. As used herein, "sexual maturation"means that an animal has acquired a reproductive ability and corresponds to the puberty in human. For example, in the case of mouse, symptom of marked cognitive abnormality appears about 50 to about 60 days after acquiring reproductive ability, and the cognitive abnormality lasts for at least about 1-2 months, preferably throughout the lifetime. However, the onset of cognitive abnormality is not particularly limited and may be before sexual maturation, as long as it is after the period when IL-1 or an analog thereof or intracellular signal transducer(s) induced by IL-1 or an analog thereof is excessively present in the body.

The cognitive abnormality in the animal model of the present invention is recognized by the presence of a significant difference from the same kind of normal animal in at least one of the conventionally known ethological measurements. The conventionally known ethological measurements include, but not limited to, measurements such as prepulse inhibition in startle response, latent inhibition, social interaction and an amount of animal activity.

The prepulse inhibition in startle response is a test for perception-response ability using a startle response, which can be commonly evaluated in human and animals, as an index. This test is characterized in that abnormalities in attention and ability to process information in brain, which are considered to be the main disease state of schizophrenia, can be scientifically and objectively evaluated. The test itself is a measurement of decrease in a fright startle response to a big sound, which is attributable to the previous application of a weak sound stimulus (prepulse) of the level incapable of causing a fright startle response, 30-150 mSec before causing a fright startle response by making a noise of about 120 db. The decrease produced by the prepulse is called a prepulse inhibition, which is known to indicate abnormal decrease in schizophrenia patients and schizophrenia animal models (Braff D.L., Geyer M.A., *Arch. Gen. Psychiatry, 47,* 181-188, 1990).

The latent inhibition is one that evaluates learning inhibition due to "habituation" before test in a series of learning tests, and the details of the test are similar to those in the aforementioned prepulse inhibition. For example, in a Pavlovian conditioning learning, getting used to bell (CS) without feeding (US) prevents learning of bell (CS)= feeding (US). Generally, schizophrenia patients and models thereof show less concentration of attention and less learning of "habituation", and therefore, they are said to be less susceptible to learning inhibition by previously presented CS or latent inhibition (Brauch I., Hemsley D.R., Gray J.A., J. Nerv. Ment. *Dis., 176,* 598-606, 1991).

The social interaction is a test capable of affording an index of the phenomenon of schizophrenia patients who dislike contact with human and withdraw from the society. In animals, smelling upon first encounter with a new animal is generally measured (Sams-Dodd F., *Rev. Neurosci., 10(1),* 59-90, 1999). The presence of the abnormality in the case of mental diseases including schizophrenia patients is generally known.

The present invention also provides an evaluation method of schizophrenia-like cognitive dysfunction using the animal model of the present invention. Said method is characterized in that the animal model of the present invention and a mammal which is same kind of the model are subjected to ethological measurements such as prepulse inhibition in startle response, latent inhibition, social interaction and an amount of animal activity, and a difference from normal animals in the cognitive ability is examined. In the animal model of the present invention, remarkable increase in prepulse inhibition is observed after sexual maturation.

The above-mentioned evaluation method can be applied to the evaluation of conventional therapeutic agents and therapeutic methods of schizophrenia, as well as the discovery of novel therapeutic agents and therapeutic methods of schizophrenia. That is, a therapeutic agent of schizophrenia can be evaluated and screened by administering a test substance to the animal model of the present invention, subsequently subjecting the model to ethological measurements such as prepulse inhibition in startle response, latent inhibition, social interaction and an amount of animal activity, and evaluating the improvement in the cognitive abnormality. In addition, the effect of a therapeutic method of schizophrenia can be evaluated by giving an external stimulus as a non-drug therapy to the animal model of the present invention, subjecting the model to ethological measurements in the same manner, and evaluating the improvement in the cognitive abnormality.

### Example

The present invention is explained in detail by referring to an example, which is a mere example and not to be construed as limiting the scope of the invention.

### Example 1 <Chronic abnormality of startle response and prepulse inhibition after IL-1α administration>

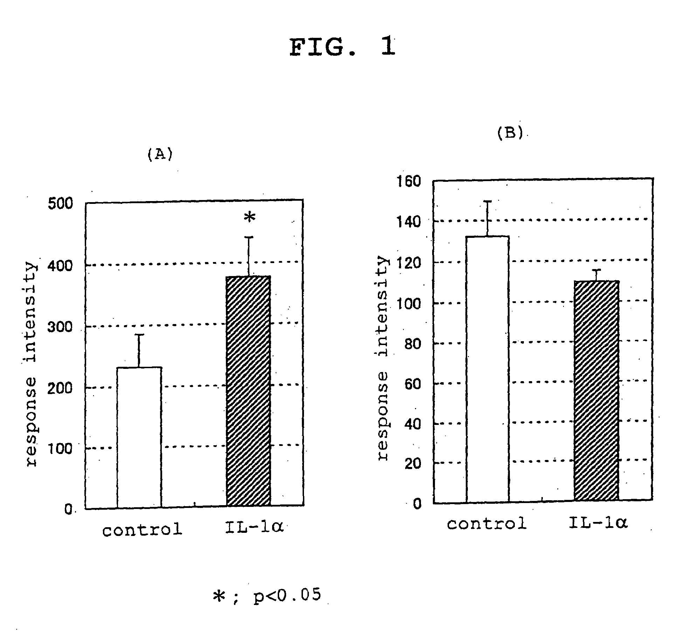

As the animal, SD rats (Japan SLC) were used from 2 days after birth. As a reagent, human recombinant interleukin 1α and cytochrome-C (Sigma) were each dissolved in physiological saline at 40 µg/mL and used. The reagents were subcutaneously administered 10 times in total (up to 11 days after birth) into the neck at 25 µL (each 1.0 mg/kg) per 1 g of rat body weight every other day from 2 days after birth. From 21 days after birth, startle response intensity and prepulse inhibition were measured with an apparatus of measuring startle response of small animal (San Diego Instruments). That is, as a sensory stimulus to induce a startle response, a sound stimulation was used, and as a prepulse stimulation, a sound pressure stimulus of 75 db was given, which was 5 db higher than the environmental noise (background noise) level. After 100 milli-seconds, a pulse stimulus of a sound pressure of 120 db was given. The ratio of decrease in the startle response when prepulse was combined to the startle response with 120 db alone was considered as the prepulse inhibition (PPI). At the time point of 60 days after birth, the IL-1α administration group showed a significant increase (Fig. 1 A) in the intensity of startle response to the sound pressure of 120 db and a significant decrease (Fig. 2A)(*p<0.05) in the prepulse inhibition, as compared to the cytochrome-C administration group. In contrast, at 22 days after birth (Fig. 1B and Fig. 2B), such abnormality was not found, and the time course pattern of the onset of schizophrenia in the puberty was same.

### Industrial Applicability

The schizophrenia-like cognitive dysfunction animal model of the present invention accurately reproduces the disease state in human as compared to conventional schizophrenia models, because it shows continuous cognitive dysfunction after sexual maturation. Using this animal model, it is possible to appropriately evaluate a therapeutic agent, a diagnostic agent or a non-drug therapeutic method of schizophrenia, and develop a novel therapeutic agent, a diagnostic agent, or a therapeutic method thereof.

This application is based on a patent application No. 52546/2001 filed on February 27, 2001 in Japan, the contents of which are hereby incorporated by reference.

## Claims

1. A mammal that is a schizophrenia-like cognitive dysfunction animal model characteristically showing continuous cognitive abnormality after sexual maturation, which is caused by an excessive presence of at least one of interleukin 1 or an analog thereof and/or intracellular signal transducers induced by interleukin 1 or an analog thereof in the body during the entire or partial stages of brain function development.

2. The mammal of claim 1, which is selected from the group consisting of mouse, rat, rabbit, guinea pig, monkey, pig, dog and cat.

3. The mammal of claim 2, which is a mouse.

4. The mammal of any of claims 1-3, wherein the interleukin 1 is interleukin 1α.

5. The mammal of any of claims 1-4, wherein said cognitive abnormality is recognized by the presence of a significant difference from the same kind of normal animal in at least one ethological measurement selected from the group consisting of prepulse inhibition in startle response, latent inhibition, social interaction and an amount of animal activity.

6. A method of preparing a schizophrenia-like cognitive dysfunction animal model characteristically showing continuous cognitive abnormality after sexual maturation, which comprises administering at least one of interleukin 1 or an analog thereof and/or intracellular signal transducers induced by interleukin 1 or an analog thereof to a mammal in an amount sufficient to cause said cognitive abnormality during the entire or partial stages of brain function development.

7. A method of preparing a schizophrenia-like cognitive dysfunction animal model characteristically showing continuous cognitive abnormality after sexual maturation, which comprises introducing an expression vector comprising a nucleic acid encoding interleukin 1 or an analog thereof into a mammal, and allowing the vector to express interleukin 1 or an analog thereof in the body of said mammal in an amount sufficient to cause said cognitive abnormality during the entire or partial stages of brain function development.

8. A method of preparing a schizophrenia-like cognitive dysfunction animal model characteristically showing continuous cognitive abnormality after sexual maturation, which comprises introducing an expression vector comprising a nucleic acid encoding a substance that induces the production of an endogenous intracellular signal transducer induced by interleukin 1 or an analog thereof, into a mammal, and allowing the vector to produce said endogenous intracellular signal transducer in the mammal body in an amount sufficient to cause said cognitive abnormality during the entire or partial stages of brain function development.

9. A mammal which is a model for a schizophrenia-like cognitive dysfunction, which can be obtained by the method of any of claims 6-8.

10. A method of evaluating a schizophrenia-like cognitive dysfunction, which comprises subjecting the mammal of any of claims 1-5 and 9 to at least one ethological measurement selected from the group consisting of prepulse inhibition in startle response, latent inhibition, social interaction and an amount of animal activity for testing for cognitive abnormality.

11. A method of screening a therapeutic agent of schizophrenia, which comprises administering a test substance to the mammal of any of claims 1-5 and 9, subsequently subjecting the mammal to at least one ethological measurement selected from the group consisting of prepulse inhibition in startle response, latent inhibition, social interaction and an amount of animal activity, and evaluating improvement in cognitive abnormality.

12. A method of evaluating a therapeutic method of schizophrenia, which comprises giving an external stimulus to the mammal of any of claims 1-5 and 9, subsequently subjecting the mammal to at least one ethological measurement selected from the group consisting of prepulse inhibition in startle response, latent inhibition, social interaction and an amount. of animal activity, and evaluating improvement in cognitive abnormality.
